# EUROPEAN PATENT APPLICATION

(11) **EP 1 881 066 A1**
(43) Date of publication of application: **23.01.2008**
(21) Application number: 06076454.5
(22) Date of filing: 21.07.2006
(51) Int. Cl.: C12N 9/02, C12P 33/00

(54) **New whole cell biocatalyst for 15ß-hydroxylation of steroids**

(71) Applicant: Nederlandse Organisatie voor toegepast- natuurwetenschappelijk onderzoek TNO, 2628 VK Delft (NL)
(72) Inventor: Ruijssenaars, Harald Johan, 3972 PL Driebergen-Rijsenburg (NL)
(74) Representative: van Loon, C.J.J.

(57) **Abstract**

The invention in its broadest sense comprises a method for enzymatic 15β-hydroxylation of steroids, wherein a cytochrome P450 steroid hydroxylase is expressed in a solvent tolerant host organism and the steroid to be hydroxylated at the 15β position is added to said organism.

## Description

The invention relates to microbial hydroxylation of steroids, more in particular to microbial systems for the 15β-hydroxylation of steroids, even more in particular to systems which use a cytochrome P450 enzyme.

The use of microbiological systems for the introduction of hydroxyl groups at sites of the steroid molecule has been known for many years, and still remains the most important method for this chemical conversion (Kieslich, K. "Steroid conversions", in: Charney, W., Herzog, H. "Microbial Transformation of Steroids", Acad. Press, New York - Zürich, 1967; Holland et al., Steroids 63, 1998, 484-495). For the 15β-hydroxylation of steroids in most cases the bacterium *Bacillus megaterium* is used, in particular the strain designated as ATCC 13368. It has been found in the literature that the enzyme *of B. megaterium* ATCC 13368 that is responsible for the conversion, is a member of the cytochrome P450 family (Berg, A. et al., 1975, Biochem. Biophys. Res. Commun. 66, 1414-1423; Berg, A. et al., 1976, J. Biol. Chem. 251, 2831-2838; Berg, A. et al., 1979, J. Biol. Chem. 254, 5264-5271). From these studies, it has appeared that the cytochrome P450 requires the presence of electron transfer components, such as a combination of an NADPH-dependent reductase and a ferredoxin, as well as NADPH and oxygen, for the hydroxylation of progesterone and other steroid substrates. The gene coding for this enzyme has been cloned and sequenced (Rauschenbach, R., et al., 1993, Mol. Gen. Genet. 241, 170-176) and was designated *cyp106A2.*

*B. megaterium* ATCC 13368 has been used frequently for providing 15β-hydroxylation of steroids (see, e.g. US 4,337,311; US 4,196,204; US 2002/0035260 and DD 266,592). However, a disadvantage of a conversion system using whole cells *of B. megaterium* ATCC 13368, is the formation of byproducts, i.e., *B. megaterium* forms other hydroxylated steroids than those produced by cell-free *cyp106A2* encoded cytochrome P450 (unpublished observations). This may imply that *B. megaterium* contains other steroid hydroxylating enzymes that can interact with the desired hydroxylation. CYP106A2 can be purified from *B. megaterium* or the enzyme can be expressed in a heterologous host (Rauschenbausch *et al., supra*). The use of (heterologously produced) purified enzyme in *in-vitro* systems, also indicated as cell-free systems, has been described in the literature. However, in this case, an electron transport chain as well as cofactors need to be provided for a proper functioning of the enzyme (Berg *et al.,* 1979, *supra;* Lisurek, M. et al., 2004, Biochem. Biophys. Res. Comm. 319, 677-682).

The present invention now offers a solution overcoming the problems that are encountered in the prior art as described above.

### Summary of the Invention

The invention relates to a method for enzymatic 15β-hydroxylation of steroids, wherein a cytochrome P450 steroid hydroxylase is expressed in a solvent tolerant host organism and adding the steroid to be hydroxylated at the 15β position to said organism. In such a method preferably components of an electron transport chain are additionally expressed in said solvent tolerant host organism, said components preferably comprising one or more of a reductase, more preferably an FAD or FMN-dependent reductase such as a flavodoxin reductase or putidaredoxin reductase. Said components also preferably comprise an electron shuttle protein, more preferably a flavodoxin or a ferredoxin, such as putidaredoxin, YkuP, YkuN or Fer.

The method of the invention preferably employs a cytochrome P450 hydroxylase derived from *Bacillus megaterium* ATCC 13368, more preferably wherein said enzyme is encoded by the gene designated as *cyp106A2,* or mutant genes thereof.

The solvent tolerant host organism of the above method preferably is a bacterium of the genus *Pseudomonas,* more preferably of the species *P. putida,* most preferably *P. putida* S12.

Preferably the steroids are selected from the group consisting of 3-keto-4-*ene*-steroids and 4-aza-steroids.

Also part of the invention is a solvent tolerant *Pseudomonas* bacterium, more preferably a *P. putada* bacterium, most preferably a *P. putida* strain S12 bacterium, which has been provided with a gene coding for a cytochrome P450 steroid hydroxylase, more preferably a cytochrome P450 steroid hydroxylase derived from *Bacillus megaterium* ATCC 13368, most preferably the cytochrome P450 steroid hydroxylase encoded by the gene designated as *cyp106A2.* Said bacterium is preferably additionally provided with one or more components or genes encoding components of an electron transport chain, preferably one or more of the components listed in above.

A further embodiment of the invention is a method for producing a solvent tolerant *Pseudomonas* bacterium according to the invention, wherein the bacterium is transformed with a gene coding for a cytochrome P450 steroid hydroxylase, more preferably a cytochrome P450 steroid hydroxylase derived from *Bacillus megaterium* ATCC 13368, most preferably with the gene designated as *cyp106A2,* and wherein the bacterium optionally is transformed with a gene coding for one or more components of an electron transport chain, preferably a gene coding for one or more of the components listed above.

Also an embodiment of the present invention is the use of a solvent tolerant bacterium according to the invention for the 15β-hydroxylation of steroids. For said use the steroids are preferably selected from the group consisting of 3-keto-4-*ene*-steroids and 4-aza-steroids.

### Legends to the Figures

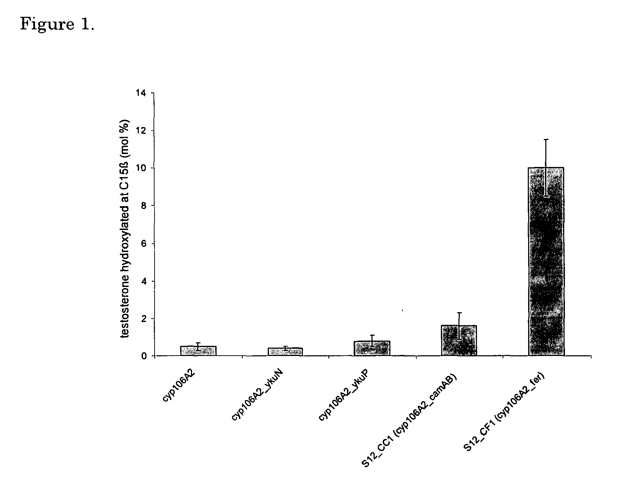

**Fig. 1.** 15β-Hydroxylation of testosterone by *P. putida* S12 expressing CYP106A2 and additional electron transfer components. Data represent conversion figures after 72 h of incubation.

### Detailed Description

Solvent tolerant bacteria are well described in literature. Preferably used are bacteria from the genus *Pseudomonas,* preferably bacteria from the species *Pseudomonas putida.* Especially *P. putida* S12 has been found to be suitable for the present invention. Use of these solvent tolerant bacteria allows for the application of a two-phase culturing system, which, next to an aqueous phase also contains an organic phase (Wierckx, N.J.P., Ballerstedt, H., De Bont, J.A.M., and Wery, J. Appl. Environ. Microbiol., 71, 8221-8227, 2005). This second phase, formed by an organic solvent, is well suited to comprise the steroid substrate for the enzymatic conversion reaction. The solvent can be a cyclic or acyclic, aromatic or non-aromatic hydrocarbon or a substituted hydrocarbon. Examples of such a solvent are linear, branched or cyclic alkanes, alkenes and alkynes, aromatic solvents, alcohols, etc. Preferred are toluene, ethylacetate or chloroform, since these are especially suited for solving the steroids which need to be supplied tot the bacterial culture. Furthermore, *P. putida* S12, in contrast to other bacterial species, is tolerant for a wide range of solvents as a result of the presence of multiple defence mechanisms that alleviate solvent stress (Wery, J., and De Bont, J.A.M. In: Ramos, J.L. (ed), Pseudomonas, vol. 3, Kluwer, Dordrecht, 2004, p. 609-634).

Methods for transforming *P. putida* S12 with heterologous genes are also known in the literature. Preferably such transformations are accomplished by using a shuttle vector, which can replicate both in *E. coli* and in *P. putida.* Preferably, this shuttle vector is the plasmid pTn-1, which was obtained by inserting a terminator downstream of the multiple cloning site (MCS) of pJWB5N (unpublished results). Plasmid pJWB5N was obtained from plasmid pUCP22 (West SEH, Schweizer HP, Dall C, Sample AK, and Runyen-Janecky LJ., Gene 55, 81-86, 1994). The DNA region *lacZa* in pUCP22 was replaced by an MCS by PCR techniques. The regulatory DNA sequence *nagR*/P*nagAa* (Husken LE, Beeftink R, de Bont JA, Wery J., Appl Microbiol Biotechnol (2001) 55 571-577) was subsequently cloned in the MCS, and behind the promoter part *P_{nagAa}* a number of cloning sites were maintained for the insertion of coding sequences.

As indicated above, the gene coding for the cytochrome P450 steroid 15β-hydroxylase is preferably the gene from *B. megaterium* ATCC 13368, *cyp106A2.* The hydroxylase encoded by this gene will hydroxylate a broad range of differently substituted 3-keto-4-*ene*-steroids, as well as 4-aza-steroids, primarily at the 15β position. More specifically the following steroid substrates are mentioned in the patent literature: pregna(di)enes (DD 266,592; US 2958631), fluoropregnadienes (US 3105795), 4-aza-steroids (US 2002/0035260; WO 9850419-A2), 1α,2α-methylene steroids (US 4,337,311; US 4196204). *B. megaterium* whole-cell systems are furthermore applied to hydroxylate steroids at the 11α-position for production of 9,11-epoxysteroids (WO 98/25948; WO9721720-A2) and for 25-hydroxylation of vitamin D-3 (JP 2003/325175-A).

The sequence of the *cyp106A2* gene is known from Rauschenbach *et al.* (*supra*). From the experiments described below it appeared that the enzyme was functionally expressed in *P. putida* S12. However, to be able to exert its 15β-hydroxylase function, also components from an electron transfer chain should be available, as described above. The biochemical components of such a transfer chain are a reductase and an electron shuttle protein. Preferably, the reductase is an enzyme which has an FAD or FMN co-factor. It will take up electrons from NAD(P)H and transfer these to the electron shuttle protein. An example of such a reductase, which is suitably used in the present invention is putidaredoxin reductase from *P. putida* ATCC 17453. However, other reductases, which are capable of taking up electrons from NAD(P)H would also be usable. For the electron shuttle protein both flavodoxins and ferredoxins can be used. Specific examples of these are putidaredoxin from *P. putida* ATCC 17453, which was shown to be an electron donor to cytochrome P450_{cam} (Peterson, J.A., 1990, J. Biol. Chem. 265, 6066-6073) and YkuP, YkuN and Fer from *Bacillus subtilis,* which have been shown to be an electron donor to the cytochrome P450 Biol (Lawson, R.J. et al., 2004, Biochemistry 43, 12390-12409; Green, A.J., 2003, J. Inorg. Biochem. 93, 92-99). However, also other electron donors which react with a cytochrome P450 enzyme could be used in the present invention.

The other components that participate in the hydroxylation reaction, such as NAD(P)H and oxygen do not specifically have to be introduced. NAD(P)H is provided through the metabolism of the solvent tolerant bacteria, and the availability of oxygen is controlled in the fermentation process. Of course, the solvent tolerant bacteria should be able to provide these components, i.e. they should be maintained in an environment that sustains their normal metabolism.

The use of solvent tolerant bacteria for the conversion of steroids has large advantages. First of all one of the major advantages are that the steroid can be applied to the bacterial culture in an organic solvent. Steroids are practically insoluble in aqueous solutions, but have a good solubility in relatively polar solvents, such as toluene, chloroform and ethyl acetate. The presence of an organic second phase also allows for a better isolation of the formed products. Further, the use of a heterologous expression system for the 15β-hydroxylase results in more selectivity, i.e. less contaminating side reactions. Compared to cell-free systems, the method of the invention is advantageous, since the heterologous host cell can provide for the components for the reduction of the cytochrome P450 enzyme. Also, the use of the heterologous host cell circumvents the need for electrochemical regeneration of cofactors or the addition of cofactor-regenerating enzyme systems including the substrates required by these systems.

The following examples will illustrate the invention in more detail, but they are not meant to limit the invention in any respect.

### Examples

### Expression of CYP106A2 in P. putida S12

The gene encoding the *B. megaterium* steroid 15β-hydroxylase, *cyp106A2,* was obtained by PCR on genomic DNA *of B. megaterium* using primers 1 and 2 (Table 2). The PCR product was cloned into the *Eco*RI and *Not*I sites of expression plasmid pTn-1 yielding pTn_cyp106A2. After transforming this plasmid to *P. putida* S12, the resulting strain hydroxylated testosterone to 15β-hydroxytestosterone. From 100 µM of testosterone, 0.7 µM of 15β-hydroxytestosterone was produced after 72 h (Fig. 1). No hydroxylated testosterone derivatives were found in cultures of *P. putida* S12 [pTn-1] (empty vector control) as well as uninduced *P. putida* S12[pTn_cyp106A2].

### Improved 15β-hydroxylation of testosterone by co-expression of CYP106A2 and the putidaredoxin / putidaredoxin reductase system in P. putida S12

*P. putida* S12[pTn_cyp106A2] was shown to hydroxylate testosterone, implying that endogenous proteins from *P. putida* S12 can transfer electrons to CYP106A2. In order to further improve the testosterone conversion, other electron transport proteins were co-expressed with CYP106A2.

Since no encoding gene sequences or amino acid sequences of the native electron transfer components megaredoxin and megaredoxin reductase are available, the P450_{cam}-supporting electron transport chain was selected for co-expression with CYP106A2. P450_{cam} is the camphor 5-exo hydroxylase of *P. putida* ATCC17453 (Poulos et al., J. Biol. Chem. 260, 1985, 16122-16130; Unger et al.,. J. Biol. Chem 261, 1986, 1158-1163). The associated electron transport chain consists of the iron-sulfur protein putidaredoxin (Pdx), and a flavo-protein, putidaredoxin reductase (PdR) (Peterson et al.,. J. Biol. Chem. 265, 1990, 6066-6073). The genes encoding P450_{cam} (*camC*), Pdx (*camB*) and PdR (*camA*) are located as *camCAB* in the camphor utilization operon of the 230-kb CAM plasmid of *P. putida* ATCC17453. This electron transport chain has been successfully applied to support both wild-type and mutant CYP119, a cytochrome P450 from *Sulfolobus solfataricus* (Koo et al., J. Am. Chem. Soc. 124, 2002, 5684-5691) that is less related to P450_{cam} than CYP106A2. Furthermore, putidaredoxin is strongly related to adrenodoxin, which acts as an electron shuttle for CYP106A2 (Berg *et al.,* 1979, *supra*)*.* The P450_{cam} electron transfer system is homologous to the expression host which is advantageous with regard to the expression of these proteins.

The 2.9-kb *camCAB* region from the *P. putida* ATCC17453 CAM plasmid was amplified by PCR and cloned into the *Eco*RI and *Not*I sites of pTn-1, yielding plasmid pTn_camCAB. Genes *camC* and *camAB* were amplified separately, using primers 4 and 5, respectively, 6 and 7, introducing a *PacI* site around the TAA stop codon of *camC* (see Table 2 for primer sequences). After transformation of pTn_camCAB to *P. putida* S12, the resulting strain was tested for camphor hydroxylation. It was demonstrated that camphor was hydroxylated, confirming the active expression of both P450_{cam} and its supporting electron transport chain.

Subsequently, the gene encoding P450_{cam} was replaced by *cyp106A2,* yielding plasmid pTn_cyp106A2_camAB. For this purpose, *cyp106A2* was amplified from genomic *B. megaterium* DNA with primers 1 and 3 (Table 2). This plasmid was transformed to *P. putida* S12 and the testosterone-hydroxylating capacity of the resulting strain was determined. The average concentration of 15β-hydroxytestosterone formed after 72 h from 100 µM of testosterone was 1.1 µM. The best performing transformant out of five tested was selected for further evaluation. This strain, designated S12_CC1, produced 2.3 µM from 100 µM testosterone after 72 h (Fig. 1). Thus, the 15β-hydroxylation of testosterone in *P. putida* S12 was improved by a factor 3 by co-expressing Pdx and PdR.

### Improved 15β-hydroxylation of testosterone by co-expression of CYP106A2 and B. subtilis electron transport proteins in P. putida S12

Rauschenbach et al. (*supra*) observed that CYP106A2 was expressed as a fully functional enzyme in *B. subtilis* strain 168. Also, CYP106A2 could be complemented with *B. subtilis* cell extract. Thus, it was concluded that a functional electron transport chain capable of supporting CYP106A2 was present in *B. subtilis.* The components of this system were, however, not identified.

From the *B. subtilis* genome (http:/genolist.pasteur.fr/SubtiList/), three putative electron shuttles were identified: Fer, a small 4Fe-4S ferredoxin and two short-chain flavodoxins YkuN and YkuP. The genes encoding Fer, YkuN and YkuP were amplified by PCR, introducing *Pac*I and *Not*I sites for cloning purposes (for primers applied: see Table 2) The *camAB*-genes from pTn_cyp 106A2_camAB were replaced by the *ykuN, ykuP* and *fer* genes. The resulting plasmids, pTn_cyp106A2_ykuN, pTn_cyp106A2_ykuP and pTn_cyp106A2_fer, were transformed to *P. putida* S12 and several transformants were tested for testosterone hydroxylation.

*P. putida* expressing CYP106A2 and YkuP produced 1 µM of 15β-hydroxytestosterone from 100 µM of testosterone, which is comparable to the strain co-expressing CYP106A2 and CamAB. Co-expression of YkuN appears to have no positive effect on the activity of CYP106A2: 0.4 µM of 15β-hydroxytestosterone was produced from 100 µM of substrate. Co-expression of Fer significantly improved the conversion of testosterone and the best performing transformant, designated strain S12_CF1, was selected for further evaluation. Strain S12_CF1 produced 10 µM of 15β-hydroxytestosterone from 100 µM of testosterone. Thus, by co-expressing Fer, the 15β-hydroxylation of testosterone by CYP106A2 was improved by a factor 14 compared to the strain without co-expressed electron transfer components.

### Modification of the product specificity of P. putida-expressed CYP106A2

The product specificity of CYP106A2 is not absolute. Progesterone is hydroxylated mainly at the 15β-position, but also on C6β, C11α and C9a (Lisurek et *al.,* supra). With *P. putida*-expressed CYP106A2, testosterone is hydroxylated mainly at C15β, but also 6β-hydroxytestosterone was observed. Co-expressed electron transfer components do not greatly affect the product spectrum (Table 3).

An active site mutant of CYP106A2 was constructed in which the threonine residue next to the conserved Thr247 was replaced by valine by PCR using mutagenic primers 14 and 15 (Table 2). The wild-type *cyp106A2* in pTn_cyp106A2_fer was replaced by the mutant gene and the resulting plasmid, pTn_cyp106A2T248V_fer was transformed to *P. putida* S12 for expression testing. The product profile of CYP106A2(T248V) with co-expressed Fer was altered with respect to the relative amount of 15β-hydroxytestosterone produced. Of the total amount of hydroxylated testosterone formed by CYP106A2(T248V), 93 % consisted of 15β-hydroxytestosterone. With wild-type CYP106A2, 75-80 % of the total amount of hydroxylated testosterone consisted of 15β-hydroxytestosterone (Table 3). Thus, the T248V active site mutation allows control of the by-product formation by *P. putida*-expressed CYP106A2.

### Tables and Figures

**Table 1. Strains and plasmids used in this study**

| strain / plasmid | relevant characteristics | source / reference |
|---|---|---|
| *E. coli* DH5a | general cloning host | Invitrogen |
| *P. putida* S12 | wild type | Hartmans et al., 1990 |
| *P. putida* DSM50198 | CAM plasmid; *camCAB* source | DSMZ |
| *Bacillus megaterium* DSM2987 | *cyp106A2* source | DSMZ |
| *Bacillus subtilis* strain 168 | *ykuN, ykuP, fer* source | DSMZ |
| pTn-1 pTn_cyp106A2 | *E. coli - P. putida* shuttle vector; *P_{nag},* Gm^{R}, Ap^{R}, pUCP22 backbone pTn-1 containing *cyp106A2* | Nijkamp et al., 2005 this study |
| pTn_camCAB | pTn-1 containing *camCAB* | this study |
| pTn_cyp106A2camAB | pTn_camCAB; *camC* replaced by *cyp106A2* | this study |
| pTn_cyp 106A2ykuN | pTn_cyp106A2camAB; *camAB* replaced by *ykuN* | this study |
| pTn_cyp106A2ykuP | pTn_cyp106A2camAB; *camAB* replaced by *ykuP* | this study |
| pTn_cyp106A2fer | pTn_cyp106A2camAB; *camAB* replaced by *fer* | this study |
| pTn_cyp106A2T248Vfer | pTn_cyp106A2fer; *cyp106A2* replaced by *cyp106A2T248V* | this study |

**Table 2. Oligonucleotides used in this study**

| Oligonucleotide primer | Target gene | Sequence (5' - 3')^{a} | Cohesive end |
|---|---|---|---|
| Primer 1 | *cyp106A2* | GCATGAATTCATGAAAGAAGTTATTGCAGTAAAAG | *Eco*RI |
| Primer 2 | *cyp106A2* | GTCTGCGGCCGCTTACATGCGGCTTGCCTTAAGCG | *Not*I |
| Primer 3 | *cyp106A2* | GCTTAATTAATTACATGCGGCTTGCCTTAAGCG | *Pac*I |
| Primer 4 | *camC* | GCATGAATTCATGACGACTGAAACCATACAAAGC | *Eco*RI |
| Primer 5 | *camC* | GCTTAATTAAACCGCTTTGGTAGTCGCCGG | *Pac*I |
| Primer 6 | *camA* | GCTTAATTAAATGGGAGTGCGTGCTAAGTGAACG | *Pac*I |
| Primer 7 | *camB* | GCATGCGGCCGCTTACCATTGCCTATCGGGAACATCG | *Not*I |
| Primer 8 | *fer* | GCTTAATTAAATCTGGGAGGTTTTATTCATGGC | *Pac*I |
| Primer 9 | *fer* | GCATGCGGCCGCGATTGAGCAGCAGCGAAGTGC | *Not*I |
| Primer 10 | *yhuN* | GCTTAATTAACAACTAATGGGGTGATAACATGGC | *Pac*I |
| Primer 11 | *ykuN* | GCATGCGGCCGCCCGATGCCGTAACAGCGGTTGC | *Not*I |
| Primer 12 | *ykuP* | GCTTAATTAAAGAGGAGGAACAAGGAAATGGCGAAG | *pac*I |
| Primer 13 | *ykuP* | GCATGCGGCCGCCCCAGTGAGTGATCAGACAGCG | *Not*I |
| Primer 14 | *cyp106A2* | GGAGTCGAGACAGTCAGTCATTTATTGG | mutagenic primer ACC-GTC |
| Primer 15 | *cyp106A2* | CCAATAAATGACTGACTGTCTCGACTCC | mutagenic primer ACC-GTC |

| | | | |
|---|---|---|---|
| a The restriction sites used for cloning have been underlined; mutated codons are in bold. | | | |

**Table 3. Regiospecificity of testosterone hydroxylation by CYP106A2 with different co-expressed electron transfer components**

| construct | 15β: 6β |
|---|---|
| pTn_cyp106A2 | 5.0 : 1 |
| pTn_cyp106A2camAB | 4.0 : 1 |
| pTn_cyp106A2ykuN | 3.0 : 1 |
| pTn_cyp106A2ykuP | 4.0 : 1 |
| pTn_cyp106A2fer | 4.2 : 1 |
| pTn_cyp106A2T248Vfer | 12.9 : 1 |

## Claims

1. Method for enzymatic 15β-hydroxylation of steroids, wherein a cytochrome P450 steroid hydroxylase is expressed in a solvent tolerant host organism and adding the steroid to be hydroxylated at the 15β position to said organism.

2. Method according to claim 1, wherein additionally components of an electron transport chain are expressed in said solvent tolerant host organism.

3. Method according to claim 2, wherein said components comprise one or more of a reductase, more preferably a FAD or FMN-dependent reductase such as a flavodoxin reductase or putidaredoxin reductase.

4. Method according to claim 2 or 3, wherein said components comprise an electron shuttle protein, more preferably a flavodoxin or a ferredoxin, such as putidaredoxin, YkuP, YkuN or Fer.

5. Method according to any of claims 1-4, wherein the cytochrome P450 hydroxylase is derived from *Bacillus megaterium* ATCC 13368, more preferably wherein said enzyme is encoded by the gene designated as *cyp106A2,* or mutant genes thereof.

6. Method according to any of claims 1-5, wherein the solvent tolerant host organism is a bacterium of the genus *Pseudomonas,* more preferably of the species *P. putida,* most preferably *P. putida* S12.

7. Method according to any of claims 1-6, wherein the steroids are selected from the group consisting of 3-keto-4-*ene*-steroids and 4-aza-steroids.

8. A solvent tolerant *Pseudomonas* bacterium, more preferably a *P. putida* bacterium, most preferably a *P. putida* strain S12 bacterium, which has been provided with a gene coding for a cytochrome P450 steroid hydroxylase, more preferably a cytochrome P450 steroid hydroxylase derived from *Bacillus megaterium* ATCC 13368, most preferably the cytochrome P450 steroid hydroxylase encoded by the gene designated as *cyp106A2.*

9. A solvent tolerant *Pseudomonas* bacterium according to claim 8, additionally provided with one or more components of an electron transport chain, preferably one or more of the components listed in claim 3 and/or claim 4.

10. Method for producing a solvent tolerant *Pseudomonas* bacterium according to claim 8 or 9, wherein the bacterium is transformed with a gene coding for a cytochrome P450 steroid hydroxylase, more preferably a cytochrome P450 steroid hydroxylase derived from *Bacillus megaterium* ATCC 13368, most preferably with the gene designated as *cyp106A2,* and wherein the bacterium optionally is transformed with a gene coding for one or more components of an electron transport chain, preferably a gene coding for one or more of the components listed in claim 3 and/or claim 4.

11. Use of a solvent tolerant bacterium according to claim 8 or 9 for the 15β-hydroxylation of steroids.

12. Use according to claim 10, wherein the steroids are selected from the group consisting of 3-keto-4-*ene*-steroids and 4-aza-steroids.
